# EUROPEAN PATENT APPLICATION

(11) **EP 4 332 987 A1**
(43) Date of publication of application: **06.03.2024**
(21) Application number: 22193775.8
(22) Date of filing: 02.09.2022
(51) Int. Cl.: G16H 50/30

(54) **COMPUTERIZED METHOD AND SYSTEM FOR DETERMINING AN EXERCISE-RELATED INDEX FOR AN EXERCISING HUMAN USER**

(71) Applicant: Eclypia, 38000 GRENOBLE (FR)
(72) Inventor: BLANC, Romain, 38000 Grenoble (FR); LE ROUX-MALLOUF, Thibault, 38000 Grenoble (FR)
(74) Representative: Fidal Innovation

(57) **Abstract**

Method for determination of an event-related index for a human user, where :
- One provides time-based data for an observation period, with :
∘ Time-based blood glucose data for the totality of the observation period, said time-based blood glucose data being derived from a continuous glucose monitor,
∘ Time-based event data for the event period,

- A computerized index determination module (1015) determines said event-related index as a composite index of the blood glucose data related to the event period and the blood glucose data outside the event period.

## Description

### FIELD OF THE INVENTION

The present invention relates to computerized methods and systems for determining an exercise-related index for an exercising human user, and associated computer programs.

More specifically, the invention relates to the monitoring of exercising human users.

### BACKGROUND OF THE INVENTION

There is a recent trend in monitoring exercising activities in humans. This trend started in competition, because data analysis is believed to be able to assist in enhancing the performance of competitors. For example, during a soccer game, individual players may be monitored. The measurements performed during the game may be post-processed. For example, the distance run by the player during the game may be calculated. These measurements, or indicators deriving therefrom may be analysed. For example, it may be possible to compare the distance run by the player during different games, and to inform the player about the game-to-game evolution of this parameter. Analysing this data may for example be used for setting a tactic for an upcoming game.

Even the average human user may have interest in monitoring their body, especially when exercising. It was recently provided electronic devices which can measure the number of steps and/or the instantaneous blood rate of an individual. Such information may be used in every day life, but are typically used by the user when exercising. There may be various reasons for performing such measurements. One reason may be health related: the user wants to confirm that, when exercising, s/he is not reaching or stepping over limits that s/he is unable to withstand. For example, a high blood rate for a long period of time may indicate a health risk. Another reason may be to quantify performance. For example, the user might consider it an improvement if s/he runs a given usual path with a decreasing maximum blood rate.

In a totally different field, diabetic people have recently started using continuous glucose monitors ("CGMs") to monitor their blood glucose concentration. "CGMs" typically provide a measurement of blood glucose every 5 minutes. These CGMs have become increasingly popular among diabetic people, because they are minimally invasive, and provide repeated measurements, which can be useful to treat diabetes by timely insulin injections.

In WO 2021/042,079, it was proposed that a cyclist uses a continuous glucose monitor so as to determine its glucose exposure. Based on a comparison of the measured glucose exposure with a threshold, the system and process may recommend the user to embark on glucose reducing or limiting actions, such as going for a walk, or eating a low carb lunch.

The present inventors have discovered that blood glucose concentration determined by a "CGM" may be very useful for assisting human users to better manage their blood glucose in relation to their exercising performance. This may be extended to other events than exercise.

### BRIEF SUMMARY OF THE INVENTION

Thus, the invention relates to a computerized method for determining an exercise-related index for an exercising human user, wherein

One provides time-based data for an observation period including an event period, said data comprising :
- Time-based blood glucose data for the totality of the observation period, said time-based blood glucose data being derived from a continuous glucose monitor,
- Time-based event data for the event period,

A computerized index determination module determines said event-related index as a composite index of the blood glucose data related to the event period and the blood glucose data outside the event period, said event-related index being determined as a function at least of the blood glucose data and the time-based event data.

Thanks to these provisions, one is able to quantify the relationship between the management of blood glucose and the lifestyle for the human user.

According to different aspects, it is possible to provide the one and / or the other of the characteristics below taken alone or in combination.

According to one embodiment, said data comprises at least one of:
- time-based pre-event data for a pre-event period just before the event period,
- time-based post-event data for a post-event period just after the event period,
- time-based start of event data for a start of event period at the start of the event period,
and wherein said computerized index determination module determines said event-related index as a function of at least one respective of the time-based pre-event data, the time-based post-event data and the time-based start of event data.

Thus, one may more precisely quantify the effect of the event.

According to one embodiment, said data further comprises night/sleep/resting data for a night/sleep/resting period within the observation period, and wherein said computerized index determination module determines said event-related index as a function further of said night/sleep/resting data.

Thus, one may more precisely quantify the effect of the event taking into account the night, sleep or resting state of the user.

According to one embodiment, said data further comprises meal data for at least one meal period within the observation period, and wherein said computerized index determination module determines said event-related index as a function further of said meal data.

Thus, one may more precisely quantify the effect of the event taking into account the meals of the user.

According to one embodiment, said computerized index determination module determines said event-related index in further view of an input data from an other sensor, in particular wherein said other sensor is not a continuous glucose monitor, and more particularly wherein the other sensor provides data representative of an effort during the event.

This enables to obtain event-specific data which may be useful to provide a more relevant event-related index.

According to one embodiment, said computerized index determination module determines said event-related index as a function of the blood glucose data during at least a first and a second periods of the observation period, wherein the first and second periods are different from one another, notably do not overlap with one another.

Thus, one is able to quantify the effect of the event on the user.

According to one embodiment, said first blood glucose parameter is one of:
- a max value of blood glucose during a time period,
- a min value of blood glucose during a time period,
- an average value of blood glucose during a time period,
- a variation of blood glucose during a time period,
- a duration of time within a time period where blood glucose satisfies a predetermined condition such as in particular being greater than or lower than a predefined value, between two predefined values.

Thus, one is able to more precisely quantify the effect of the event on the user.

According to one embodiment, wherein said first blood glucose parameter is one of the variation of blood glucose concentration between the first and second part of the night; the ratio of time spent during day-time below a predetermined threshold of, for example 90 milligram per decilitre of blood (mg/dl); the variability of blood glucose during day-time; the minimum value of blood glucose during an outside meal time interval during the day; the maximum value of the blood glucose during the day; the ratio of time spent below a predetermined threshold of, for example 90 milligram per decilitre of blood (mg/dl) during the observation period; the ratio of time spent during day-time of the current day below a predetermined threshold of, for example 90 milligram per decilitre of blood (mg/dl); the minimum value of blood glucose during an outside meal time interval during daytime for a predefined number (greater than 1, typically 7) of the latest days; the variability of blood glucose during the observation period; the maximum value of the blood glucose during the observation period; the ratio of time spent below a predetermined threshold of, for example 90 milligram per decilitre of blood (mg/dl) for a predefined number (greater than 1, typically 7) of the latest days; when a meal is classified as high carbonated, the variation of blood glucose concentration during a post-prandial time interval; when a meal is classified as low carbonated, the variation of blood glucose concentration during a post-prandial time interval; the minimum value of blood glucose during a time interval which comprises during exercising and after exercising; the minimum value of blood glucose during a post-exercising time interval or a minimum value of a slope of the blood glucose during this post-exercise time interval; the minimum value of blood glucose during a time interval which comprises during exercising; the maximum value of blood glucose during a pre-exercising time interval of a predetermined duration, for example one hour; in case of a max blood glucose exceeding a predetermined threshold of, for example, 150 mg/dl during a pre-exercising time interval of a predetermined duration of, for example one hour, a minimum value of blood glucose during this same post-exercising time interval after this peak; the minimum value of variability of blood glucose during a pre-exercising time interval of a predetermined duration, for example one hour; a predetermined range of blood glucose level around an average level for example between 90 and 130 mg/dl outside which there are measurements for more than a predetermined amount of time of, for example, five minutes, during a pre-exercising time interval of a predetermined duration of, for example thirty minutes; a predetermined range of blood glucose level around an average glucose level of for example between 90 and 130 mg/dl outside which there are measurements during a predetermined time of, for example, at least one measurement, during a pre-exercising time interval of a predetermined duration of, for example ten minutes; the variation of blood glucose during a start of exercise time interval of a predetermined duration, for example 30 minutes; the variation of blood glucose during a start of exercise time interval of a predetermined duration, for example 30 minutes in case of an average negative slope as determined from data obtained by an external meter; variability of the blood glucose measurement taking into account the intensity of the exercise, as determined by input data from external sensor such as, for example, slope of the ground, power developed by the user, etc... ; the variation of blood glucose during the exercising time interval; the variation of blood glucose during the exercising time interval; the average of blood glucose during the exercising time interval; the minimum of blood glucose during a post-exercising time interval of less than a predetermined amount of time of, for example, one hour; the variation of blood glucose during a post-exercising time interval of less than a predetermined amount of time of, for example, one hour.

Thus, one is able to precisely label a glycemic event occurring at a user.

According to one embodiment, said computerized index determination module determines said event-related index as a function of the blood glucose data associated with one or more of the above categories: rest/night/sleep blood glucose; average blood glucose during daytime; average blood glucose during the observation period; post-meal blood glucose; exercise-related hypoglycemia outside the exercise time period itself; blood glucose during a predefined time before the exercise; blood glucose during a pre-defined time just before the exercise; blood glucose at the start of the exercise; blood glucose reactivity at the start of the exercise; blood glucose reactivity during the exercise; blood glucose reactivity just after the exercise.

According to one embodiment, said computerized index determination module determines said event-related index as a function of a plurality of marks each associated to a respective blood glucose related parameter, wherein said mark is determined by comparison of a value of the blood glucose related parameter with a threshold scale.

Thus, one is able to compare and aggregate on a common scale a variety of different blood glucose related parameters.

According to one embodiment, said function sums said plurality of marks, and in particular weighted marks, where the weights relate to the relevancy of the parameter for the index.

Thus, one provides a non-computationally intense index.

According to one embodiment, said first and second blood glucose related parameters are determined for the same or for different periods of the observation period.

Thus, one is able to obtain various relevant information from the relevant time periods of an observation period.

According to an embodiment, the observation period is of at least 12 hours, notably at least 24 hours.

According to an embodiment, the observation period is of at most 72 hours, notably at most 48 hours.

According to an embodiment, the event period is of at least 30 minutes, notably at least one hour, in particular at least 2 hours, or even at least 4 hours.

According to an embodiment, the event comprises exercising, and in particular an endurance effort such as at least running, cycling or swimming.

The above method appears more properly suited in these conditions.

According to an embodiment, time-based event data is received from user declaration and/or detection of user activity through an activity sensor.

According to a further aspect, the invention relates to a computerized method for monitoring the performance of an exercising human user, wherein said computerized index determination module repeatedly determines an exercise-related index for an exercising human user by the above computerized method.

According to a further aspect, the invention relates to computer program comprising instructions to cause a processor to execute the steps of any of the above methods.

According to a further aspect, the invention relates to a computerized system for determining an event-related index for a human user, comprising a computerized index determination module adapted to determine said event-related index as a composite index of blood glucose data related to an event period and blood glucose data not related to the event period, said event-related index being determined as a function at least of:
- time-based blood glucose data for the totality of the observation period, said time-based blood glucose data being derived from a continuous glucose monitor, and
- time-based event data for the event period.

### BRIEF DESCRIPTION OF THE DRAWINGS

Embodiments of the invention will be described below with reference to the drawings, described briefly below:
Fig. 1 represents a human user wearing a sensing system.
Fig. 2 is a schematic diagram representing a wearable sensing system according to an embodiment.
Figs. 3-5 are screen shots of a user interface enabling a user to enter event information.
Fig. 6 is a diagrammatic view of a processor of a sensing system.
Fig. 7 is a schematic diagram representing a wearable sensing system according to another embodiment.
Fig. 8 is a screenshot of a user interface showing monitoring a composite index.

In the drawings, identical references designate identical or similar objects.

### DETAILED DESCRIPTION OF THE INVENTION

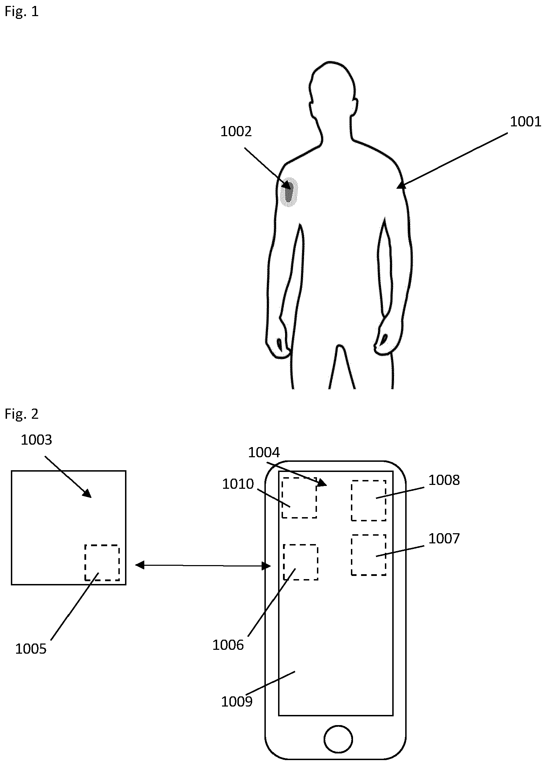

Fig. 1 represents a human user 1001 wearing a wearable sensing system 1002. The wearable sensing system can be placed in contact or close proximity to the skin of the human user 1001. It may be worn in any suitable location, such as at the back of the upper arm, the distal part of the forearm, the dorsal face of the wrist, on the abdomen or elsewhere. The wearable sensing system 1002 is adapted to be worn by the user during a given duration of time. After that time, the wearable sensing system 1002 may be replaced. Typically, the time during which the wearable sensing system is worn is of at least one hour. It may typically be of at least 24 hours, at least 48 hours, at least 96 hours, etc...

### Data acquisition

In particular, the wearable sensing system 1002 comprises a continuous glucose monitor. Various continuous glucose monitors exist on the market. A continuous glucose monitor determines concentration of blood glucose "continuously", i.e. at repeated short intervals of time. In view of the kinetics of blood glucose variation in humans, an interval of time of less than ten minutes is generally considered as short enough for the glucose monitor to be "continuous". A typical interval of time is of, for example, 5 minutes, between two subsequent measurements. In addition, the measurement is performed "automatically", in that the human user (or any one else) does not need to perform any specific action for the measurement to be performed. Hence, every few minutes, a continuous glucose monitor provides a measurement of blood glucose concentration G.

Typically, the continuous glucose monitor comprises a clock, so that the continuous glucose monitor repeatedly provides vectors (Gᵢ, tᵢ), with i an integer indicia ranging from 1 to n, where n is an integer, where Gᵢ describes the measured blood glucose concentration, and tᵢ the time associated with the measurement.

The wearable system 1002 comprises a user interface. According to an embodiment, the wearable system 1002 comprises a single device encompassing the continuous glucose monitor and the user interface. However, according to other embodiments, such as shown for example on Fig. 2, the wearable system 1002 may comprise a wearable device 1003 and a remote electronic device 1004. This architecture enables notably the wearable device to remain compact, which can be useful for social reasons in everyday life, but also to assist in the wearable device remaining firmly attached to the body, and an electronic device dedicated to various functions, including user interfaces.

Under this architecture, the wearable system 1002 comprises a communication system which comprises a wearable communication module 1005 at the wearable device 1003 and a remote communication module 1006 at the remote electronic device 1004, the wearable communication module 1005 and remote communication module 1006 being adapted to communicate with one another to exchange information in one or both directions. This communication may be wireless, such as by RFID, NFC, or under a Bluetooth^{™} protocol available at the earliest priority date of the present document.

The wearable system continues to be considered wearable as long as at least one of its components can be worn.

The wearable system 1002, and in particular the electronic device 1004 comprises a data storage 1007, where data can be stored. The electronic device 1004 may comprise the clock 1008, so that data which are stored in the data storage include both glucose concentration as determined by wearable device 1003 and time, determined by the clock 1008, at which the concentration is received by the electronic device 1004.

As discussed above, the electronic device 1004 comprises a user interface 1009, adapted to receive information from the user and/or to provide information to the user. In the present case, the user interface 1009 will be described as a touch screen. However, other embodiments are possible, such as through mechanical buttons, LEDs, microphone, voice recognition and loud speakers or else.

The electronic device 1004 comprises a processor 1010 adapted to operate the other electronic components of the device 1004.

The user interface 1009 allows the user to enter information to the wearable sensing system 1002. This information is stored in the data storage 1007.

For example, the processor 1010 executes a computer program which enables the user to enter information into the wearable sensing system 1002 such as through windows enabling to enter text using a keyboard or the like.

In particular, the information may be entered live or a posteriori.

If the information is entered live, it is associated with the instant time as defined by the clock 1008. If the information is entered a posteriori, the entered information also comprises time related to the information.

The user interface 1009 is for example used for entering event information. In the description below, one embodiment will be detailed when the event is a sporting event, i.e. the user exercises during a given time. However, the invention is equally applicable to other events, such as, for example, travelling, performing arts, driving, working, taking an exam, or many other events which require energy from the user.

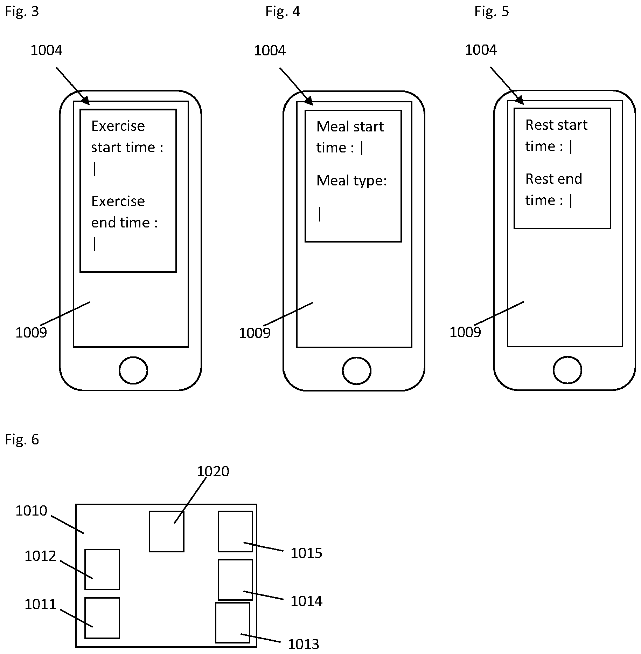

As can be seen for example on Fig. 3, the user interface 1009 enables the user to enter exercise start time tₑ₁ and exercise end time tₑ₂.

For example, after exercising, the user will use the user interface 1009 to enter the exercise start time tₑ₁ and the exercise end time tₑ₂.

Alternatively, when starting to exercise, the user will use the user interface 1009 to provide the information that he is starting to exercise, and the exercise start time tₑ₁ will be provided by the clock. At the end of the exercise, the user will use the user interface 1009 to provide the information that he is stopping to exercise, and the exercise end time tₑ₂ will be provided by the clock 1008. Alternatively, tₑ₁ and tₑ₂ are obtained from a training management software in communication with the wearable sensing system.

The data storage 1007 also comprises an observation start time tₒ₁ and an observation end time tₒ₂. For example, the clock 1008 provides time upon replacement of a wearable system 1002. In this example, the observation start time tₒ₁ is defined as the time of starting a new wearable system, and the observation end time tₒ₂ is defined as the time of stopping using this wearable system.

According to another example, the observation start time tₒ₁ is determined as a predefined amount of time before the exercise start time tₑ₁ (for example 60 min to 180 min before tₑ₁) and the observation end time tₒ₁ is determined as a predefined amount of time after the exercise end time tₑ₂ (for example 60 min to 300 min after tₑ₂). For example, one obtains data for a longer period of time and, a posteriori, in view of a number of parameters, such as the evolution of blood glucose, tₒ₁ and tₒ₂ are determined. In particular, the observation start time and the observation end time are defined with respect to a single event one wishes to analyse, sufficiently before and after this single event. If the user wears a continuous glucose monitor for a time substantially longer than this observation period associated with a single event, typically 10 days or more, measurement data may be continuously obtained for a time covering a plurality of events, and a plurality of observation periods, for example one associated with each event, maybe defined.

The data storage 1007 also comprises a meal start time tₘ₁ and a meal end time tₘ₂. As can be seen for example on Fig. 4, the user interface 1009 enables the user to enter meal start time tₘ₁. For example, the user will enter a time at which he expects to eat, or at which he ate. Alternatively, the user may not enter time, but use the user interface 1009 to inform that he is starting a meal. The clock 1008 will then provide the meal start time tₘ₁ as the instantaneous time. Meal end time tₘ₂ may be determined as a predetermined time after meal start time tₘ₁. Alternatively, meal start and/or end time may be detected automatically from blood glucose data and/or movement detection sensors.

The data storage 1007 might also comprise data associated with the meal. For example, data may comprise a meal type, such as defined in a predefined list comprising {breakfast, lunch, dinner, snack}, and/or a qualification of the meal, such as {low carb/high carb}. This may be entered by the user using the user interface 1009. Alternatively, this may be determined automatically, for example by a computerized analysis of one or more pictures of the meal. The limit between low and high carbonated meals may be parametered and/or predefined such as, for example, to 100 g of carbo-hydrates.

The data storage 1007 also comprises a rest start time tᵣ₁ and a rest end time tᵣ₂. As can be seen for example on Fig. 5, the user interface 1009 enables the user to enter a rest start time tᵣ₁ and a rest end time tᵣ₂. Since it might be difficult for some users to determine a time at which they actually fall asleep, "rest", "sleep" or "night" are considered identical in the present discussion. The system does not need to detect night based on ambient light measurements, nor sleep based on physiological signals. For example, the rest start time tᵣ₁ and the rest end time tᵣ₂ are predefined.

### Time interval determination

As can be seen on Fig. 6, the processor 1010 implements a time interval determination module 1011 which is adapted to determine time intervals from the data.

For example, the time interval determination module 1011 determines a time interval from the stored time data.

For example, the time interval determination module 1011 may comprise night-related time interval determination modules.

For example, a start of night time interval determination module determines a start of night interval as starting at tᵣ₁, and ending at a time tᵣ₃ before tᵣ₂. For example, tᵣ₃ is determined so that the start of night interval lasts for a predetermined duration of, for example, four hours.

According to another example, tᵣ₃ may be set as an average of tᵣ₁ and tᵣ₂.

For example, an end of night time interval determination module determines a end of night interval as ending at tᵣ₂, and starting at a time tᵣ₄ after tᵣ₁. For example, tᵣ₄ is determined so that end of night interval lasts for a predetermined duration of, for example, four hours.

According to another example, tᵣ₄ may be set as an average of tᵣ₁ and tᵣ₂.

According to some examples, tᵣ₃ = tᵣ₄.

According to some examples, the start of night interval and the end of night interval may overlap, or may not overlap.

The time interval determination module 1011 may comprise a day time interval determination module. For example, it is determined that all times of the observation period which are not in the night time interval are in a day time interval. Hence, a start of day time t_{d1} is set equal to an end of night time tᵣ₂. An end of day time td2 is set equal to a start of night time tᵣ₁.

Alternatively to what is disclosed above, the time interval determination module 1011 may be adapted to determine the night time intervals from the day time intervals.

For example, the time interval determination module 1011 may comprise meal-related time interval determination modules.

For example, a post-prandial time interval determination module determines a post-prandial time interval as starting at tₘ₂, and ending a predetermined time after tₘ₂, such as, for example, in particular, 90 minutes after tₘ₂.

For example, an outside meal time interval determination module determines an outside meal time interval as starting at a time tₘ₃, for example a predetermined amount of time after tₘ₂, in particular 90 minutes after tₘ₂, and ending a predetermined time after tₘ₃, such as, for example, in particular, 60 minutes after tₘ₃.

For example, the time interval determination module 1011 may comprise exercise-related time interval determination modules.

For example, a post-exercise time interval determination module determines a post-exercise time interval as starting at tₑ₂, and ending a predetermined time after tₑ₂, such as, for example 60 to 300 minutes after tₑ₂.

According to one embodiment, if a meal takes place after the exercise during the observation period, the end of the post-exercise time interval is set to the start time of this meal. According to one embodiment, the post-exercise time interval determination module may determine a short and a long post-exercise time intervals, both starting at tₑ₂, and ending at different times, where the short post-exercise time interval is shorter than the long post-exercise time interval. For example, the short post-exercise time interval may last for 45-90 minutes. For example, the long post-exercise time interval may last for 120 to 300 minutes.

For example, a pre-exercise time interval determination module determines a pre-exercise time interval as ending at tₑ₁, and starting a predetermined time before tₑ₁, such as, for example one hour before tₑ₁, and such as 30 minutes before tₑ₁.

For example, a start of exercise time interval determination module determines a start of exercise time interval as starting at tₑ₁, and ending at a time tₑ₃ before tₑ₂. For example, tₑ₃ is determined so that start of exercise time interval lasts for a predetermined duration of, for example, one hour or thirty minutes.

Alternatively, tₑ₃ may be determined also from tₑ₂, so that the start of exercise time interval lasts for a predetermined portion of the exercise time interval, for example about 10% of the duration of the exercise time interval.

As described above, the time interval determination module 1011 is used to determine time intervals. Such time intervals will typically last for at least 15 minutes. Some of these time intervals may be partially overlapping with one another.

During each of these intervals, one or more values of blood glucose are measured by the continuous glucose monitor.

The processor 1010 operates a blood glucose treatment module 1012 so as to treat blood glucose data during determined time intervals.

If a determined time interval lasts for at least 15 minutes, and if a continuous glucose monitor provides measurement data every five minutes, there are at least 3 measurement points in each time interval. There may be much more measurement points in one or more time intervals.

### Blood glucose data treatment

The blood glucose treatment module 1012 may operate one or more of the following functions on the blood glucose data.

Mean/Averaging: The "mean/averaging" function for a time interval provides an average of the blood glucose data in that time interval.

Time spent below a threshold: The "time spent below a threshold" function for a time interval identifies subsequent blood glucose measurements below a threshold, and determines the difference between the time of the last one of these measurements and the first one of these measurements. If there are a plurality of time sub-intervals with blood glucose measurements below a threshold, separated by blood glucose measurements above this threshold, the "time spent below a threshold" function sums the durations of the plurality of time sub-intervals with blood glucose measurements below the threshold. In case it is not null, the time spent below the threshold is at least equal to the data acquisition sampling time.

Time spent above a threshold: The "time spent above a threshold" function for a time interval identifies subsequent blood glucose measurements above a threshold, and determines the difference between the time of the last one of these measurements and the first one of these measurements. If there are a plurality of time sub-intervals with blood glucose measurements above a threshold, separated by blood glucose measurements below this threshold, the "time spent above a threshold" function sums the durations of the plurality of time sub-intervals with blood glucose measurements above the threshold. In case it is not null, the time spent above the threshold is at least equal to the data acquisition sampling time.

Time spent in range: The "time spent in range" function for a time interval identifies subsequent blood glucose measurements in range below a higher threshold and above a lower threshold, and determines the difference between the time of the last one of these measurements and the first one of these measurements. If there are a plurality of time sub-intervals with blood glucose measurements in range, separated by blood glucose measurements out of range, the "time spent in range" function sums the durations of the plurality of time sub-intervals with blood glucose measurements in range. In case it is not null, the time spent in range is at least equal to the data acquisition sampling time.

Ratio of time spent: Time spent below/above a threshold or in range may be determined as described above. The ratio of time spent is determined as this value divided by the total duration of the time interval.

Min: The "min" function for a time interval provides the minimum of the blood glucose values during that time interval.

Max: The "max" function for a time interval provides the maximum of the blood glucose values during that time interval.

Variation: The "variation" function for a time interval provides the difference between the last blood glucose value for the time interval, and the first blood glucose value for the time interval.

Further, the blood glucose treatment module 1012 may operate one or more of the following functions to compare blood glucose values associated with two different time intervals.

Increase/Decrease: The "increase/decrease" function provides a difference between two predetermined blood glucose values. Depending on the sign of the result, the difference is considered as an increase or a decrease. Each predetermined blood glucose value is associated with a respective time interval. It is for example, the min, the max, the mean, the first or the last value associated with this interval.

Area under the curve:_The "area under the curve" function provides an area of a surface bound by blood glucose value curve, a horizontal threshold (potentially equal to 0), and two vertical lines corresponding to the start and the end of the time interval.

Cost function: The "cost" function is a predefined function of a plurality of blood glucose values. The "cost" may be related to a feature of the curve one wishes to detect. For example, a "cost" function may be predefined to provide a high resulting value in case of hypoglycemia and/or hyperglycemia, and a low value in the absence thereof. One example is a so-called Boiroux cost function.

Standard deviation: The "standard deviation" function provides the square root of the variance of the blood glucose data within the interval.

Variation coefficient: The "variation coefficient" function provides the ratio of the "standard deviation" and the average, as both defined above.

Curve length: The "curve length" function determines the length of the curve passing by the glucose data points during the time interval.

Peak: The "peak" function determines the existence, and possibly the intensity of a peak in the blood glucose data, i.e. two subsequent inverse variations of the blood glucose data during a time interval. For example, the "peak" function may determine a minimum value for the blood glucose, a blood glucose data point close to average closest before the minimum value, a blood glucose data point close to average closest after the minimum value, and an area under the curve between these two closest points, the curve and a threshold defined by these two closest points.

Time spent above, below a slope threshold or in slope range: These functions are similar to the time spent above, below a threshold or in range as defined above, but using the slope of the glucose rather than the absolute value.

Variability: This function determines a variability of the curve, or of part of the curve. Many mathematical operators are applicable, such as for example defined in Schall et al., "la variabilité glycémique en reanimation" in Annales françaises d'anesthesie et de reanimation (Vol. 31, No. 12, pp. 950-96°), Elsevier-masson, in particular in Annex 1, hereby incorporated by reference to define "variability" according to some embodiments.

These functions may be applied to the raw blood glucose data as provided by the continuous glucose monitor. However, it is also possible that the raw blood glucose data are pre-processed and/or filtered before applying the above functions.

### Parameter evaluation

The processor 1010 comprises a computerized parameter evaluation module 1013. The parameter evaluation module 1013 processes time-based blood glucose data and time-based event data, such as time-based exercise data, time-based rest/sleep/night data, time-based meal data, in order to provide a numerical value associated with a predefined parameter.

The parameter evaluation module 1013 associates a time interval with blood glucose data measured during this interval. Hence, the parameter evaluation module 1013 filters out blood glucose data outside the considered time interval.

According to some examples, the parameter evaluation module 1013 may also use as input data coming from another sensor. For example, input data may relate to the local slope of the ground the user is on. This input data may for example be determined from a positioning sensor of the user, enabling to determine his position and movement, and a database comprising information as to the slope of the ground in that location. Alternatively, a digital inclinometer may provide information about the slope of the ground at the location of the user without global positioning. Other embodiments are possible.

According to another example of input data from another sensor, one may process power input data representative of the instantaneous power produced by the user. Such input data is for example provided by a powermeter, for example uses in the biking industry. Other kinds of input data related to the instantaneous power produced buy the user are possible.

The above two examples are just two examples of external data usable by the parameter evaluation module 1013 together with the blood glucose and event data, but other embodiments are possible.

A first category of parameters is associated with rest/night/sleep blood glucose.

The first parameter relates to the variation of blood glucose concentration between the first and second part of the night.

The parameter evaluation module 1013 determines the first parameter P1 as the variation of glycemia between a start of night interval and an end of night interval.

A second category of parameters is associated with average blood glucose during daytime.

The parameter evaluation module 1013 determines the second parameter P2 as the ratio of time spent during day-time below a predetermined threshold of, for example 90 milligram per decilitre of blood (mg/dl).

The parameter evaluation module 1013 determines the third parameter P3 as the variability of blood glucose during day-time.

The parameter evaluation module 1013 determines the fourth parameter P4 as the minimum value of blood glucose during an outside meal time interval during daytime.

The parameter evaluation module 1013 determines the fifth parameter P5 as the maximum value of the blood glucose during daytime.

The parameter evaluation module 1013 determines the twenty-sixth parameter P26 as the ratio of time spent during day-time of the latest day below a predetermined threshold of, for example 90 milligram per decilitre of blood (mg/dl).

The parameter evaluation module 1013 determines the twenty-seventh P27 as the minimum value of blood glucose during an outside meal time interval during daytime for a predefined number (greater than 1, typically 7) of the latest days.

A third category of parameters is associated with average blood glucose during the observation period.

The parameter evaluation module 1013 determines the sixth parameter P6 as the ratio of time spent below a predetermined threshold of, for example 90 milligram per decilitre of blood (mg/dl) during the observation period.

The parameter evaluation module 1013 determines the seventh parameter P7 relates to the variability of blood glucose during the observation period.

The parameter evaluation module 1013 determines the eighth parameter P8 as a maximum value of the blood glucose during the observation period.

The parameter evaluation module 1013 determines the twenty-eighth parameter P28 as the ratio of time spent below a predetermined threshold of, for example 90 milligram per decilitre of blood (mg/dl) for a predefined number (greater than 1, typically 7) of the latest days.

A fourth category of parameters is associated with post-meal blood glucose.

The parameter evaluation module 1013 determines the ninth parameter P9 as, when a meal is classified as high carbonated, the variation of blood glucose concentration during a post-prandial time interval.

The parameter evaluation module 1013 determines the tenth parameter P10 as, when a meal is classified as low carbonated, the variation of blood glucose concentration during a post-prandial time interval.

A fifth category of parameters is associated with exercise-related hypoglycemia outside the exercise time period itself.

The parameter evaluation module 1013 determines the eleventh parameter P11 as a minimum value of blood glucose during a time interval which comprises during exercising and after exercising.

The parameter evaluation module 1013 determines the twelfth parameter P12 as a minimum value of blood glucose during a post-exercising time interval or a minimum value of a slope of the blood glucose during this post-exercise time interval.

The parameter evaluation module 1013 determines the twenty-ninth parameter P29 as a minimum value of blood glucose during a time interval which comprises during exercising.

A sixth category of parameters is associated to blood glucose during a predefined time before the exercise.

The parameter evaluation module 1013 determines the thirteenth parameter P13 as a maximum value of blood glucose during a pre-exercising time interval of a predetermined duration, for example one hour.

The parameter evaluation module 1013 determines the fourteenth parameter P14 as, in case of a max blood glucose exceeding a predetermined threshold of, for example, 150 mg/dl during a pre-exercising time interval of a predetermined duration of, for example one hour, a minimum value of blood glucose during this same post-exercising time interval after this peak.

The parameter evaluation module 1013 determines the fifteenth parameter P15 as a minimum value of variability of blood glucose during a pre-exercising time interval of a predetermined duration, for example one hour.

A seventh category of parameters is associated to blood glucose during a pre-defined time just before the exercise.

The parameter evaluation module 1013 determines the sixteenth parameter P16 as a predetermined range of blood glucose level around an average glucose level of for example between 90 and 130 mg/dl outside which there are measurements during a predetermined time of, for example, 5 minutes, during a pre-exercising time interval of a predetermined duration of, for example thirty minutes.

An eighth category of parameters is associated to blood glucose at the start of the exercise.

The parameter evaluation module 1013 determines the seventeenth parameter P17 as a predetermined range of blood glucose level around an average glucose level of for example between 90 and 130 mg/dl outside which there are measurements during a predetermined time of, for example, at least one measurement, during a pre-exercising time interval of a predetermined duration of, for example ten minutes.

A ninth category of parameters is associated to blood glucose reactivity at the start of the exercise.

The parameter evaluation module 1013 determines the eighteenth parameter P18 as a variation of blood glucose during a start of exercise time interval of a predetermined duration, for example 30 minutes.

The parameter evaluation module 1013 determines the nineteenth parameter P19 as a variation of blood glucose during a start of exercise time interval of a predetermined duration, for example 30 minutes in case of an average negative slope as determined from data obtained by an external meter.

A tenth category of parameters is associated to blood glucose reactivity during the exercise.

The parameter evaluation module 1013 determines the twentieth parameter P20 as a variability of the blood glucose measurement taking into account the intensity of the exercise, as determined by input data from external sensor such as, for example, slope of the ground, power developed by the user, etc....

The parameter evaluation module 1013 determines the twenty-first parameter P21 as the variation of blood glucose during the exercising time interval.

The parameter evaluation module 1013 determines the twenty-second parameter P22 as the variation of blood glucose during the exercising time interval.

The parameter evaluation module 1013 determines the twenty-third parameter P23 as the average of blood glucose during the exercising time interval.

An eleventh category of parameters is associated to blood glucose reactivity just after the exercise.

The parameter evaluation module 1013 determines the twenty-fourth parameter P24 as the minimum of blood glucose during a post-exercising time interval of less than a predetermined amount of time of, for example, one hour.

The parameter evaluation module 1013 determines the twenty-fifth parameter P25 as the variation of blood glucose during a post-exercising time interval of less than a predetermined amount of time of, for example, one hour.

The above are examples of parameters which may be determined by the parameter evaluation module 1013 based on blood glucose data and event-related data. The parameter evaluation module 1013 may be adapted to determine other parameters based on these data.

### Marking module

The processor 1010 comprises a computerized marking module 1014.

The computerized marking module 1014 is adapted to compare the value determined for a parameter by the parameter evaluation module 1013 with a predefined scale, and to provide a mark for this parameter. The predefined scale may be predefined once for all, or may for example be user specific. The mark is for example a Boolean. Alternatively, the mark might be an integer or a real number. Importantly, the ranges of marks for the different parameters are comparable with one another. The predefined scale may for example comprise one single threshold. For example, the computerized marking module 1014 may determine the mark for a parameter as being equal to 0 if the value for the parameter is less than the single threshold, and as being equal to n if the value for the parameter is over the single threshold.

To the contrary, the computerized marking module 1014 may determine the mark for a parameter as being equal to n if the value for the parameter is less than the single threshold, and as being equal to 0 if the value for the parameter is over the single threshold.

"n" may for example be equal to 1. All parameters may have equal weight. However, alternatively, some parameters may be set different marks, such as for example n=1, n=2 or n=3 for various parameters.

According to another example, the predefined scale may comprise a lower threshold and an upper threshold.

For example, the computerized marking module 1014 may determine the mark for a parameter as being equal to 0 if the value for the parameter is less than the lower threshold, and as being equal to 1 if the value for the parameter is between the lower threshold and the upper threshold, and as being equal to 2 if the value for the parameter is over the upper threshold.

To the contrary, the computerized marking module 1014 may determine the mark for a parameter as being equal to 2 if the value for the parameter is less than the lower threshold, and as being equal to 1 if the value for the parameter is between the lower threshold and the upper threshold, and as being equal to 0 if the value for the parameter is over the upper threshold.

According to another option, depending on the parameter, the computerized marking module 1014 may determine the mark for a parameter as being equal to 1 if the value for the parameter is less than the lower threshold, and as being equal to 0 if the value for the parameter is between the lower threshold and the upper threshold, and as being equal to 1 if the value for the parameter is over the upper threshold.

To the contrary, the computerized marking module 1014 may determine the mark for a parameter as being equal to 0 if the value for the parameter is less than the lower threshold, and as being equal to 1 if the value for the parameter is between the lower threshold and the upper threshold, and as being equal to 0 if the value for the parameter is over the upper threshold.

The scale may comprise more than two predefined thresholds, so that different marking possibilities might be implemented depending on the parameter of interest.

Regarding the first parameter P1, the computerized marking module 1014 may set the first mark M1 to 1 if the decrease is over a predefined first threshold T1 chosen, for example, between 5 and 50 mg/dl, and to set the first mark M1 to 0 else.

Regarding the second parameter P2, the computerized marking module 1014 may set the second mark M2 to 1 if the time ratio is over a predefined second threshold T2 chosen, for example, between 10 and 50%, and to set the second mark M2 to 0 else.

Regarding the third parameter P3, the computerized marking module 1014 may set the third mark M3 to 1 if the CV is less than a predefined third threshold T3 chosen, for example, between 10 and 40, and to set the third mark M3 to 0 else.

Regarding the fourth parameter P4, the computerized marking module 1014 may set the fourth mark M4 to 1 if the minimum value is less than a predefined fourth threshold T4 chosen, for example, between 70 and 100 mg/dl, and to set the fourth mark M4 to 0 else.

Regarding the fifth parameter P5, the computerized marking module 1014 may set the fifth mark M5 to 1 if the maximum is less than a predefined fifth threshold T5 chosen, for example, between 130 and 200 mg/dl, and to set the fifth mark M5 to 0 else.

Regarding the sixth parameter P6, the computerized marking module 1014 may set the sixth mark M6 to 1 if the time ratio is over a predefined sixth threshold T6 chosen, for example, between 10% and 50%, and to set the sixth mark M6 to 0 else.

Regarding the seventh parameter P7, the computerized marking module 1014 may set the seventh mark M7 to 1 if the CV is less than a predefined seventh threshold T7 chosen, for example, between 10 and 40, and to set the seventh mark M7 to 0 else.

Regarding the eighth parameter P8, the computerized marking module 1014 may set the eighth mark M8 to 1 if the maximum is less than a predefined eighth threshold T8 chosen, for example, between 130 and 200 mg/dl, and to set the eighth mark M8 to 0 else.

Regarding the ninth parameter P9, the computerized marking module 1014 may set the ninth mark M9 to 1 if the variation is less than a predefined ninth threshold T9 chosen, for example, between 10 and 60 mg/dl, and to set the ninth mark M9 to 0 else.

Regarding the tenth parameter P10, the computerized marking module 1014 may set the tenth mark M10 to 1 if the variation is more than a predefined tenth threshold T10 chosen, for example, between 30 and 100 mg/dl, and to set the tenth mark M10 to 0 else.

Regarding the eleventh parameter P11, the computerized marking module 1014 may set the eleventh mark M11 to 1 if the minimum is less than a predefined eleventh threshold T11 chosen, for example, between 70 and 100 mg/dl, and to set the eleventh mark M11 to 0 else.

Regarding the twelfth parameter P12, the computerized marking module 1014 may set the twelfth mark M12 to 1 if the minimum is less than a predefined twelfth threshold T12 chosen, for example, between 70 and 100 mg/dl, and to set the twelfth mark M12 to 0 else.

Regarding the thirteenth parameter P13, the computerized marking module 1014 may set the thirteenth mark M13 to 1 if the maximum is over a predefined thirteenth threshold T13 chosen, for example, between 140 and 200 mg/dl, and to set the thirteenth mark M13 to 0 else.

Regarding the fourteenth parameter P14, the computerized marking module 1014 may set the fourteenth mark M14 to 1 if the minimum is below a predefined fourteenth threshold T14 chosen, for example, between 70 and 100 mg/dl, and to set the fourteenth mark M14 to 0 else.

Regarding the fifteenth parameter P15, the computerized marking module 1014 may set the fifteenth mark M15 to 1 if the CV is less than a predefined fifteenth threshold T15 chosen, for example, between 10 and 40, and to set the fifteenth mark M15 to 0 else.

Regarding the sixteenth parameter P16, the computerized marking module 1014 may set the sixteenth mark M16 to 1 if a time exceeds five minutes further away from the predefined average value than a predefined sixteenth threshold T16 chosen, for example, between 0 and 25 mg/dl, and to set the sixteenth mark M16 to 0 else.

Regarding the seventeenth parameter P17, the computerized marking module 1014 may set the seventeenth mark M17 to 1 if there are measurements further away from the predefined value than a predefined seventeenth threshold T17 chosen, for example, between 0 and 25 mg/dl, and to set the seventeenth mark M17 to 0 else.

Regarding the eighteenth parameter P18, the computerized marking module 1014 may set the eighteenth mark M18 to 1 if the variation is more than a predefined eighteenth threshold T18 chosen, for example, between 10 and 60 mg/dl, and to set the eighteenth mark M18 to 0 else.

Regarding the nineteenth parameter P19, the computerized marking module 1014 may set the nineteenth mark M19 to 1 if the variation is more than a predefined nineteenth threshold T19 chosen, for example, between 5 and 50 mg/dl, and to set the nineteenth mark M19 to 0 else.

Regarding the twentieth parameter P20, the computerized marking module 1014 may set the twentieth mark M20 to 1 if the variability is less than a predefined twentieth threshold T20 chosen, for example, between 10 and 40, and to set the twentieth mark M20 to 0 else.

Regarding the twenty-first parameter P21, the computerized marking module 1014 may set the twenty-first mark M21 to 1 if the variation is more than a predefined twenty-first threshold T21 chosen, for example, between 5 and 60 mg/dl, and to set the twenty-first mark M21 to 0 else.

Regarding the twenty-second parameter P22, the computerized marking module 1014 may set the twenty-second mark M22 to 1 if the variation is more than a predefined twenty-second threshold T22 chosen, for example, between 10 and 60 mg/dl, and to set the twenty-second mark M22 to 0 else.

Regarding the twenty-third parameter P23, the computerized marking module 1014 may set the twenty-third mark M23 to 1 if the average is more than a predefined twenty-third threshold T23 chosen, for example, between 90 and 140 mg/dl, and to set the twenty-third mark M23 to 0 else.

Regarding the twenty-fourth parameter P24, the computerized marking module 1014 may set the twenty-fourth mark M24 to 1 if the min is less than a predefined twenty-fourth threshold T24 chosen, for example, between 70 and 100 mg/dl, and to set the twenty-fourth mark M24 to 0 else.

Regarding the twenty-fifth parameter P25, the computerized marking module 1014 may set the twenty-fifth mark M25 to 1 if the variation is more than a predefined twenty-fifth threshold T25 chosen, for example, between 10 and 80 mg/dl, and to set the twenty-fifth mark M25 to 0 else.

Regarding the twenty-sixth parameter P26, the computerized marking module 1014 may set the twenty-sixth mark M26 to 1 if the time ratio is over a predefined twenty-sixth threshold T26 chosen, for example, between 10 and 50%, and to set the twenty-sixth mark M26 to 0 else.

Regarding the twenty-seventh parameter P27, the computerized marking module 1014 may set the twenty-seventh mark M27 to 1 if the minimum value is less than a predefined twenty-seventh threshold T27 chosen, for example, between 70 and 100 mg/dl, and to set the twenty-seventh mark M27 to 0 else.

Regarding the twenty-eighth parameter P28, the computerized marking module 1014 may set the twenty-eighth mark M28 to 1 if the time ratio is over a predefined twenty-eighth threshold T28 chosen, for example, between 10 and 50%, and to set the twenty-eighth mark M28 to 0 else.

Regarding the twenty-ninth parameter P29, the computerized marking module 1014 may set the twenty-ninth mark M29 to 1 if the minimum is less than a predefined twenty-ninth threshold T29 chosen, for example, between 70 and 100 mg/dl, and to set the twenty-ninth mark M29 to 0 else.

As can be understood from the discussion above, the computerized marking module 1014 may award a mark by comparison of the value of a parameter with a predefined scale. The mark itself is set within a marking scale extending between a minimum mark and a maximum mark for this parameter. The marking scales for the parameters are similar.

### Exercise-related index

The processor 1010 comprises an index determination module 1015.

The index determination module 1015 is adapted to determine an index based on the stored data. In particular, the index is a composite index of the blood glucose data related to the exercising period and the blood glucose data outside the exercising period of the user.

According to one example, the composite index is a function of two or more of the above parameters. The function is set depending on the physiological marker for which one wishes to provide an indicator. In particular, the composite index is a function of the value of these parameters with respect to a meaningful threshold. Said differently, the index determination module 1015 determines the composite index as a function of the marks attributed by the computerized marking module 1014 for the various parameters. Further, for a given index, the marks may be weighted, so as to attribute more relevance to some parameters relative to other parameters. The weights may be predefined for a given composite index. According to another example, the weight be depend of another input data such as, for example, the time of day of the event. More particularly, for a given composite index, this index is determined as a ratio of marks associated with a plurality of relevant parameters to the highest possible sum of the marks. The marks are determined based on whether a parameter reaches a threshold predetermined for this composite index. The marks may also be weighted according to the importance of the parameter for the physiological marker.

Below, one will provide a plurality of examples of a composite index determined by the index determination module 1015.

According to some examples, the composite index is an index of an insufficient nutritional intake before the event.

### Example 1: Composite index of the nutrition strategy of the user before the season start

The index above is particularly used during a training session of several days, for example before the start of a season of competition. Below is a table showing the determination of this composite index for a user within an observation period comprising an exercising time interval and a not exercising time interval.

| Parameter | Value | Threshold Tᵢ | Mark Mᵢ | Index |
|---|---|---|---|---|
| P1 | <T1 | T1 | 0/4 | 6/13=0,46 |
| P26 | >T2 | T2 | 2 | |
| P27 | <T27 | T27 | 1 | |
| P28 | <T28 | T28 | 0/3 | |
| P29 | <T29 | T29 | 2 | |
| P12 | <T12 | T12 | 1 | |

The composite index of 0,46 is an indicator of the relevance of the nutrition strategy during this period. It is determined based on a combination of parameters and scales which are relevant to the nutrition strategy during this period.

As can be seen in particular, the composite index is determined a function of parameters related to the exercise itself, and parameters not-related to the exercise itself.

As can be seen in particular, the composite index is determined a function of parameters determined for different periods of the observation period. These various periods may even not be juxtaposed nor overlap.

The value of the composite index might be used by a nutritionist, coach or physician in order to adapt the nutrition of the user in view of future exercising events.

### Example 2: Composite index of the nutrition strategy of the user during a season break

The index above is particularly used during a resting period, for example during a break within a season of competition. Below is a table showing the determination of this composite index for a user within an observation period comprising an exercising time interval and a not exercising time interval.

| Parameter | Value | Threshold Tᵢ | Mark Mᵢ | Index |
|---|---|---|---|---|
| P1 | 30 | 35 | 4 | 6/10=0,6 |
| P26 | 60 | 50 | 2 | |
| P27 | 70 | 75 | 0/1 | |
| P28 | 18 | 25 | 0/3 | |

The composite index of 0,6 is an indicator of the relevance of the nutrition strategy during that period. It is determined based on a combination of parameters and scales which are relevant to the nutrition strategy during that period.

As can be seen in particular, the composite index is determined a function of parameters related to the exercise itself, and parameters not-related to the exercise itself.

As can be seen in particular, the composite index is determined a function of parameters determined for different periods of the observation period. These various periods may even not be juxtaposed nor overlap.

The value of the composite index might be used by a nutritionist, coach or physician in order to adapt the nutrition of the user in view of future exercising events.

### Example 3: Composite index of the nutrition strategy of the user during a training session

The index above is particularly used during a single training session. Below is a table showing the determination of this composite index for a user within an observation period comprising an exercising time interval and a not exercising time interval.

| Parameter | Value | Threshold Tᵢ | Mark Mᵢ | Index |
|---|---|---|---|---|
| P29 | >T29 | T29 | 0/2 | 1/3=0.33 |
| P12 | <T12 | T12 | 1 | |

The composite index of 0,33 is an indicator of the relevance of the nutrition strategy during this period. It is determined based on a combination of parameters and scales which are relevant to the nutrition strategy during this period.

As can be seen in particular, the composite index is determined a function of parameters related to the exercise itself, and parameters not-related to the exercise itself.

As can be seen in particular, the composite index is determined a function of parameters determined for different periods of the observation period. These various periods may even not be juxtaposed nor overlap.

The value of the composite index might be used by a nutritionist, coach or physician in order to adapt the nutrition of the user in view of future exercising events.

### Example 4: Composite index of the nutrition strategy of the user before the season start

This example builds on Example 1, where the index determination module determines the time of day of the exercising event. For example, the index determination module determines that the exercising event took place in the morning, and attributes the weight to parameter P1 to 4.

According to this example, the index determination module determines the time of the exercising event to be in the afternoon, and attributes less importance to parameter "P1". The weight of this parameter is set to 3. Below is a table showing the determination of this composite index for a user within an observation period comprising an exercising time interval and a not exercising time interval.

| Parameter | Value | Threshold Tᵢ | Mark Mᵢ | Index |
|---|---|---|---|---|
| P1 | 10 | 15 | 0/3 | 6/12=0,5 |
| P26 | 45 | 25 | 2 | |
| P27 | 88 | 100 | 1 | |
| P28 | 28 | 35 | 0/3 | |
| P29 | 80 | 95 | 2 | |
| P12 | 80 | 100 | 1 | |

The composite index of 0,5 is an indicator of the relevance of the nutrition strategy during this period if the exercising event takes place in the afternoon. It is determined based on a combination of parameters and scales which are relevant to the nutrition strategy during this period.

According to this example, the index determination module determines the time of the exercising event to be in the evening, and attributes less importance to parameter "P1". The weight of this parameter is set to 2. Below is a table showing the determination of this composite index for a user within an observation period comprising an exercising time interval and a not exercising time interval.

| Parameter | Value | Threshold Tᵢ | Mark Mᵢ | Index |
|---|---|---|---|---|
| P1 | 40 | 45 | 0/2 | 6/11=0,55 |
| P26 | 33 | 35 | 2 | |
| P27 | 78 | 80 | 1 | |
| P28 | 18 | 25 | 0/3 | |
| P29 | 80 | 95 | 2 | |
| P12 | 90 | 100 | 1 | |

The composite index of 0,55 is an indicator of the relevance of the nutrition strategy during this period if the exercising event takes place in the evening. It is determined based on a combination of parameters and scales which are relevant to the nutrition strategy during this period.

As can be seen in particular, the composite index is determined a function of parameters related to the exercise itself, and parameters not-related to the exercise itself.

As can be seen in particular, the composite index is determined a function of parameters determined for different periods of the observation period. These various periods may even not be juxtaposed nor overlap.

The value of the composite index might be used by a nutritionist, coach or physician in order to adapt the nutrition of the user in view of future exercising events.

As can be seen in particular, the composite index is determined a function of parameters related to the exercise itself, and parameters not-related to the exercise itself.

As can be seen in particular, the composite index is determined a function of parameters determined for different periods of the observation period. These various periods may even not be juxtaposed nor overlap.

The value of the composite index might be used by a nutritionist, coach or physician in order to adapt the nutrition of the user in view of future exercising events.

### Example 5: Composite index of pre-/post-exercising strategy of the user

Below is a table showing the determination of this composite index for a user within an observation period comprising an exercising time interval and a not exercising time interval.

| Parameter | Value | Threshold Tᵢ | Mark Mᵢ | Index |
|---|---|---|---|---|
| P13 | 185 | 150 | 1 | 4/11=0,36 |
| P14 | 70 | 90 | 2 | |
| P17 | [0; 15] | [-25; 25] | 0/2 | |
| P19 | 0 | 5 | 0/3 | |
| P23 | 140 | 100 | 0/2 | |
| P24 | 85 | 90 | 1 | |

The composite index of 0,36 is an indicator of the relevance of the pre-/post- exercising strategy. It is determined based on a combination of parameters and scales which are relevant to the pre-/post- exercising strategy.

The value of the composite index might be used by a coach in order to ensure an optimal physiological state during a given period to avoid hypoglycemic events or inadequate nutritional intake.

The value of the composite index might be used by a coach in order to define the optimal nutritional strategy during the race for each riders or athlete.

Example 6: Composite index of pre-/post-exercising strategy of the user for a cycling event starting with a way down

Below is a table showing the determination of this composite index for a user within an observation period comprising an exercising time interval and a not exercising time interval.

| Parameter | Value | Threshold Tᵢ | Mark Mi | Index |
|---|---|---|---|---|
| P13 | >T13 | T13 | 1 | |
| P14 | <T14 | T14 | 2 | 4/8=0,5 |
| P17 | [-5; 10] | [-15; 15] | 0/2 | |
| P23 | >T23 | T23 | 0/2 | |
| P24 | <T24 | T24 | 1 | |

The composite index of 0,5is an indicator of the relevance of the pre-/post-exercising strategy when cycling starting with a way down. It is determined based on a combination of parameters and scales which are relevant to the pre-/post- exercising strategy.

The value of the composite index might be used by a coach in order to ensure an optimal physiological state during a given period to avoid hypoglycemic events or inadequate nutritional intake.

The value of the composite index might be used by a coach in order to define the optimal nutritional strategy during the race for each riders or athlete.

Other examples may be provided. For example, one determines a composite index of the effects of the training.

More particularly, depending on the training, and the expected effect of the training, some more specific indexes may be determined in this category.

For example, one index is an index of the effect of a "training the gut" training. Such "training the gut" training is a training performed in order to train the body to assimilate carbohydrates. This index may be based on two or more of the parameters P20, P21, P22 and P23. For example, this index is based on P20 and P21, on P20 and P22, on P20 and P23, on P21 and P22, on P21 and P23, on P22 and P23, on P20, P21 and P22, on P20, P21 and P23, on P21, P22 and P23, or on all of P20, P21, P22 and P23. These parameters are associated with adapted thresholds and weights, as described above. Other parameters may also additionally be used to determine this index.

For example, one index is an index of the effect of an intense training session. This index may be based on two or more of the parameters P16, P17, P20, P21, P22, and P23. For example, this index may be based on two of these parameters. Alternately, this index may be based on three, four or five of these parameters. This index may be based on all six of these parameters. These parameters are associated with adapted thresholds and weights, as described above. Other parameters may also additionally be used to determine this index.

For example, one index is an index of the effect of an endurance training session. This index may be based on two or more of the parameters P11 or P29, P12, P16, P17, P20, P21, P22, and P23. For example, this index may be based on two of these parameters. Alternately, this index may be based on three, four, five, six or seven of these parameters. This index may be based on all eight of these parameters. These parameters are associated with adapted thresholds and weights, as described above. Other parameters may also additionally be used to determine this index.

Of course, the composite index will be adapted in view of the physiological marker to be evaluated. For example, when preparing for a violin concerto by violin playing sessions or a 6-hour long written exam by sitting exam preparation sessions, the slope of the ground will be of limited relevancy.

The system which was described above may be repeated for a plurality of observation periods. Hence, a given composite index may be determined for each of a plurality of observation periods. This may enable the user to monitor the evolution of his/her composite index. This is particularly relevant if the observation periods comprise similar exercising events. For example, a composite index may be monitored if the user performs a weekly biking tour, for example on Sunday mornings, and determines the value of the composite index for each of these tours. Alternatively, this could be applied also to running and/or swimming. If the user changes his nutritional habits or pre-/post- exercising habits, these changes may be reflected in the values of the composite index.

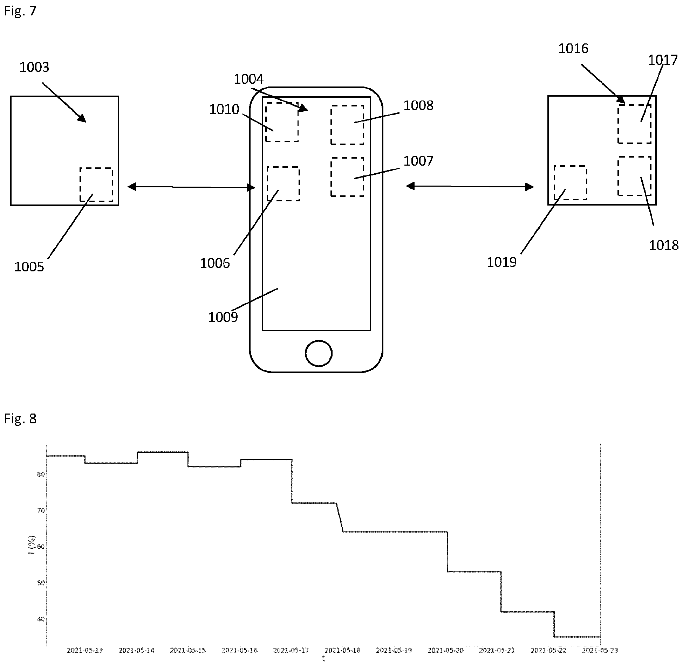

The processor 1010 hence comprises an index monitoring module 1020, which may store, display or analyze the time evolution of one or more composite index. As shown for example on Fig. 8, a composite index such as the ones described above is repeatedly determined for a user during a general period lasting from May 12^{th} 2021 to May 23^{rd}, 2021, during which a user is ongoing an intense training comprising one or more training sessions per day, for example in view of participating to an endurance sport event, such as the Mont-Blanc ultra-trail (UTMB^{®}) or cycling Tour de France^{®}. The determined value for the index is plotted as ordinate, and time as abscissa. The curve shows the evolution of the composite index along time, and shows a first part which is relatively constant and high, and a second part which is continuously decreasing from May 17^{th}, 2021. The curve may be used by the user or trainer to correlate with a potential change of training or nutrition which may have occurred before May 17^{th}, 2021, and the effect of which is witnessed by the decrease of the value of the composite index.

According to the above-described example, the processor 1010 which determines the composite index is located on the electronic device 1004 of the user. According to another architecture, as shown on Fig. 7, the system comprises a server 1016 which is remote from the electronic device 1004. The server 1016 comprises a processor 1017, a data storage 1018, and a communication module 1019 adapted to communicate with the electronic device 1004, for example with the communication module 1006 of the electronic device 1004.

For example, the communication between the electronic device 1004 and the server 1016 may comprise a wired communication. At the end of the exercising period, the electronic device may be plugged to the server 1016 or an intermediate personal computer through a suitable cable, and the stored data may be transferred from the electronic device 1004 to the server 1016. The communication may alternatively be wireless. It may imply a long-distance radio communication, such as for example using a wi-fi protocol available at the earliest priority date of the present document. Typically, data is transferred from the electronic device 1004 to the server 1016 after the end of an observation period.

The processor 1017 of the server 1016 thus comprises the time interval determination module 1011, the blood glucose treatment module 1012, the parameter evaluation module 1013, the marking module 1014, the index determination module 1015 and the index monitoring module 1020, as described above, so as to determine one or more relevant composite index for the user.

According to one example, the server 1016 may be in communication with more than one remote electronic devices 1004, these electronic devices belonging to different users. Hence, the server 1016 may be operated by a coach, and determine one or more relevant composite index for any of a plurality of users.

The system which was just described may be operated as follows.

Typically, the user is wearing a wearable device 1003 such as a continuous glucose monitor which regularly wirelessly communicates with an electronic device 1004, such as a smartphone, a value of a blood glucose measurement.

The user determines an observation period. The observation period is typically of at least 12 hours, notably at least 24 hours. The observation period may last until the wearable device is replaced. It may last typically up to 48 or 72 hours. This observation period encompasses at least an exercising period, one or more meal periods and one or more rest/sleep/night periods. Typically, the exercising period is sufficiently long in order to obtain a sufficient number of blood glucose measurements. It may last at least 30 minutes, 60 minutes, 2 hours or even 4 hours.

Upon eating, the user communicates meal data through the user interface.

Upon sleeping/resting, the user communicates sleep/rest/night data through the user interface.

The user communicates exercising data through the user interface. Alternatively, the exercising event is detected by a dedicated activity sensor in communication with the electronic device, such as a heart rate sensor and/or an accelerometer.

All events are associated with a time by the clock 1008.

At the end of the observation period, the user determines a composite index to be determined for the past or ending observation period. The composite index is selected among a plurality of available composite index. For example, the composite index was already selected by the user upon previous uses of the system, and is currently monitored.

The value for the composite index for the observation period is determined. This value may be compared to past values of this same composite index determined in the past.

This value, or evolution, may be used by a professional in order to provide exercise-related advice to the user.

All computerized functions which are defined here may be defined in computer programs which, when executed by processors cause the processor to implement the function.

While exemplary embodiment of the invention has been described with reference to a plurality of embodiments, it will be understood by those skilled in the art that various changes, omissions and/or additions may be made, and equivalents may be substituted for elements thereof without departing from the spirit and scope of the invention. In addition, many modifications may be made to adapt a particular situation or material to the teachings of the invention without departing from the scope thereof. Therefore, it is intended that the invention is not limited to the particular embodiment disclosed as the best mode contemplated for carrying out this invention, but that the invention will include all embodiments falling within the scope of the appended claims. Moreover, unless specifically stated any use of the terms first, second, etc. do not denote any order or importance, but rather the terms first, second, etc. are used to distinguish one element from another.

Some of the parameters above, some of the marks above, and/or some of the composite index above appear original per se, independently of the presently claimed invention, and the applicant reserves any right to file a divisional patent application for one or more of those, independently of the originally claimed invention.

### LIST OF REFERENCE SIGNS

- 1001 :: Human user
- 1002:: wearable sensing system
- 1003:: wearable device
- 1004:: electronic device
- 1005:: wearable communication module
- 1006:: remote communication module
- 1007:: data storage
- 1008:: clock
- 1009:: user interface
- 1010:: processor
- 1011:: time interval determination module
- 1012:: blood glucose treatment module
- 1013:: parameter evaluation module
- 1014:: marking module
- 1015:: index determination module
- 1016:: server
- 1017:: processor
- 1018:: data storage
- 1019:: communication module
- 1020:: index monitoring module

## Claims

1. Computerized method for determining an event-related index for a human user, wherein :
- One provides time-based data for an observation period including an event period, said data comprising :
∘ Time-based blood glucose data for the totality of the observation period, said time-based blood glucose data being derived from a continuous glucose monitor,
∘ Time-based event data for the event period,
- A computerized index determination module (1015) determines said event-related index as a composite index of the blood glucose data related to the event period and the blood glucose data outside the event period, said event-related index being determined as a function at least of the blood glucose data and the time-based event data.

2. Computerized method according to claim 1, wherein said data comprises at least one of:
. time-based pre-event data for a pre-event period just before the event period,
. time-based post-event data for a post-event period just after the event period,
. time-based start of event data for a start of event period at the start of the event period,
and wherein said computerized index determination module (1015) determines said event-related index as a function of at least one respective of the time-based pre-event data, the time-based post-event data and the time-based start of event data.

3. Computerized method according to claim 1 or 2, wherein said data further comprises night/sleep/resting data for a night/sleep/resting period within the observation period, and wherein said computerized index determination module (1015) determines said event-related index as a function further of said night/sleep/resting data.

4. Computerized method according to any of claims 1 to 3, wherein said data further comprises meal data for at least one meal period within the observation period, and wherein said computerized index determination module (1015) determines said event-related index as a function further of said meal data.

5. Computerized method according to any of claims 1 to 4, wherein said computerized index determination module (1015) determines said event-related index in further view of an input data from an other sensor, in particular wherein said other sensor is not a continuous glucose monitor, and more particularly wherein the other sensor provides data representative of an effort during the event.

6. Computerized method according to any of claims 1 to 5, wherein said computerized index determination module (1015) determines said event-related index as a function of the blood glucose data during at least a first and a second periods of the observation period, wherein the first and second periods are different from one another, notably do not overlap with one another.

7. Computerized method according to any of claims 1 to 6, wherein said computerized index determination module (1015) determines said event-related index as a function of at least one first blood glucose related parameter and one second blood glucose related parameter, the first and second blood glucose-related parameters being different from one another.

8. Computerized method according to claim 7, wherein said first blood glucose parameter is one of:
. a max value of blood glucose during a time period,
. a min value of blood glucose during a time period,
. an average value of blood glucose during a time period,
. a variation of blood glucose during a time period,
. a duration of time within a time interval where blood glucose satisfies a predetermined condition such as in particular being greater than or lower than a predefined value, or between two predefined values; or a ratio of this duration of time to the duration of said time interval.

9. Computerized method according to any of claims 7 to 8, wherein said computerized index determination module (1015) determines said event-related index as a function of a plurality of marks each associated to a respective blood glucose related parameter, wherein said mark is determined by comparison of a value of the blood glucose related parameter with a threshold scale.

10. Computerized method according to claim 9, wherein said function sums said plurality of marks, notably weighted marks.

11. Computerized method according to claim 6 and further according to any of claims 7 to 10, wherein said first and second blood glucose related parameters are determined for the same or for different periods of the observation period.

12. Computerized method according to any of claims 1 to 11, wherein time-based event data is received from user declaration and/or detection of user activity through an activity sensor.

13. Computerized method for monitoring the performance of a human user, wherein said computerized index determination module (1015) repeatedly determines an event-related index for a human user by the computerized method of any of claims 1 to 12.

14. A computer program comprising instructions to cause a processor to execute the steps of any of the methods of claims 1 to 13.

15. Computerized system for determining an event-related index for a human user, comprising a computerized index determination module (1015) adapted to determine said event-related index as a composite index of blood glucose data related to an event period and blood glucose data outside the event period, said event-related index being determined as a function at least of:
- time-based blood glucose data for the totality of the observation period, said time-based blood glucose data being derived from a continuous glucose monitor, and
- time-based event data for the event period.
